# EUROPEAN PATENT APPLICATION

(11) **EP 2 859 863 A1**
(43) Date of publication of application: **15.04.2015**
(21) Application number: 14275138.7
(22) Date of filing: 20.06.2014
(51) Int. Cl.: A61B 19/00, A61B 19/02, A61M 25/01

(54) **Surgical apparatus container assembly**

(30) Priority: 11.10.2013 GB 201318040
(71) Applicant: Cook Medical Technologies LLC, Bloomington, IN 47404 (US)
(72) Inventor: Dela, Christian, 2500 Valby (DK); Lundsteen, Nicholas, 4330 Hvalsoe (DK)
(74) Representative: Williams Powell

(57) **Abstract**

A surgical apparatus container assembly (30) includes a base or container element (32) and a cover (34) able to slide over the base element (32). The cover (34) includes a rounded elongated channel (50) extending along its top surface, bounded by a first and second rounded ridges (52). The base element (32) has a top surface with a shape consistent with the shape of the inside surface of the cover (34). The channel (50) provides a support and guide surface for an introducer assembly or other device held within the assembly (30) and is particularly useful in keeping an introducer assembly in the correct orientation during the course of a medical procedure, for instance aligned with a percutaneous entry point into a patient. The packaging assembly (30) can be placed, in one embodiment, between the patient's legs (106) with the cover (34) resting on the patient's thighs. This enables the support channel (50) to be located adjacent the percutaneous entry point (104).

## Description

### Technical Field

The present invention relates to a surgical apparatus container assembly for containing surgical apparatus, both for storage and transportation and also for use during a medical procedure. The preferred embodiment is directed to a container assembly for holding femoral operation apparatus.

### Background Art

Surgical apparatus, such as endoluminal introducer assemblies or treatment tools, is typically stored in tailor made transport and storage packaging for protecting the equipment and also for maintaining it sterilised until the point of use. For more complex or delicate apparatus, the packaging generally comprises a rigid or semi-rigid casing having a base portion provided with a plurality of recesses or chambers sized and shaped to hold specific components of the equipment individually. These protect the elements of the apparatus and also facilitate their retrieval during the medical procedure.

In most endoluminal surgical procedures the medical apparatus comprises a plurality of components which are used at different times, such as guide wires, introducer catheters, and implantable medical device carriers or medical tools. These components are typically found in introducer apparatus. Furthermore, even in cases where all the components are used at the same time, the introduction procedure requires staged guiding of the introducer apparatus from a remote percutaneous entry point through the patient's vasculature until the distal end of the apparatus has reached the treatment site. In the course of the medical procedure itself, it is sometimes necessary for the clinician to release grip on the surgical apparatus, for instance to carry out another medical procedure or process.

For many endoluminal procedures, the apparatus has a significant length, particularly relative to its diameter, as well as being flexible so as to be able to curve through the patient's vasculature.

A problem with such procedures is that the apparatus can often be unwieldy to manipulate, particularly having regard to keeping those parts of the apparatus which remain outside the patient in position and properly aligned with the respect to the percutaneous entry point. In cases where the apparatus must be left for a while, there is often no convenient place to rest the apparatus while the patient is on the operating table.

There have been attempts in the art to provide suitable packaging for surgical apparatus, examples being disclosed in US-5,165,540, US-5,334,142, US-5,392,918, US-2008/272,023, US-2011/186,456 and US-2011/284,410.

### Disclosure of the Invention

The present invention seeks to provide an improved surgical apparatus container assembly and in the preferred embodiments able to mitigate at least some of the disadvantages indicated above.

According to an aspect off the present invention, there is provided a surgical apparatus container assembly including a container element provided with a plurality of receptacles for accommodating surgical apparatus; and a cover shaped to fit over the container element and to close said receptacles, the cover including an upper surface forming an outer surface of the assembly, the upper surface including an elongate channel extending at least partially along the upper surface and being open at an end of the upper surface; the elongate channel being rounded or tapering in transverse cross-section.

The elongate channel is located at the top of the apparatus and in practice provides a support and guide for the surgical apparatus. In particular, and as described and shown in detail below, the elongate channel is able to hold the part of the surgical apparatus outside of the patient supported and aligned with the percutaneous entry point. For instance, the proximal end of an introducer assembly, to which the operation handle is fitted, can be rested on the container assembly, specifically within the elongate channel and thus in a set position. By orienting the channel towards the percutaneous entry point, the proximal end of the introducer assembly can be held properly aligned in position, allowing the surgeon to let go of the apparatus without risk of it moving out of position.

Advantageously, the elongate channel extends along the entire of the upper surface and is open at both of opposing ends of the assembly.

In a preferred embodiment, the cover has a length which extends in a direction along a longitudinal axis of the assembly, the elongate channel extending substantially parallel to or at an angle to said longitudinal axis. This arrangement makes it easier to provide for convenient placing and orientation of the proximal end of an introducer assembly, for instance during a femoral access operation, where the container assembly can be placed between a patient's legs.

Preferably, the cover includes a lower surface, which lower surface is provided with one or more elongate channels extending at least partially along the lower surface. In an embodiment, the cover includes two elongate channels extending at least partially along the lower surface, advantageously being disposed either side of the elongate channel in the upper surface. The or each elongate channel may be rounded in transverse cross-section. It is preferred that the or each elongate channel is sized to accommodate a part of a patient's body, such as to rest on a patient's leg.

This construction has a very useful property of enabling the over to be rested on a patient's legs, with the lower channels lying on the patient's legs and hence holding the cover in position, with the elongate upper channel properly oriented towards the femoral percutaneous entry point.

The cover may be formed from sheet material having a generally uniform thickness.

Preferably, the container element has an upper surface, which upper surface has a shape conforming to or similar to the upper surface of the cover. The container element thus can be formed just of two parts.

Advantageously, the cover is slidable over the container element. In this embodiment, the assembly may include guide elements on the cover and container element for guiding the cover in sliding manner over the container element and shaped to prevent pulling apart of the cover and container element from one another. Preferably, the guide elements include rounded depending edges to the cover and corresponding rounded side walls to the container element. Rounded parts to the container assembly can prevent or reduce the risk of tearing of any sterile wrapping over the assembly and reduce the risk of damage to components held in the container assembly should this be hit or dropped, for example.

Preferably the cover is slidable over the container element substantially in the direction of the elongate channel.

Advantageously, the cover and container element are sealable to one another, thereby to seal the receptacles.

According to another aspect of the present invention, there is provided a method of deploying surgical apparatus container by use of an assembly which includes a container element provided with a plurality of receptacles for accommodating surgical apparatus; and a cover shaped to fit over the container element and to close said receptacles, the cover including an upper surface forming an outer surface of the assembly, the upper surface including an elongate channel extending at least partially along the upper surface and being open at an end of the upper surface; the elongate channel being rounded or tapering in transverse cross-section; the method including the steps of:
placing the assembly adjacent a patient; and
disposing a medical tool in the elongate channel of the upper surface of the cover in a direction pointing to the patient.

Preferably, the cover includes two elongate channels extending at least partially along the lower surface, and sized to accommodate a part of a patient's body, the method including the step of disposing the cover on the patient's body and disposing a medical tool in the elongate channel of the upper surface in a direction pointing to the patient while the cover is on the patient's body.

Advantageously, the elongate channels are sized to rest on a patient's leg and the cover is disposed with the elongate channel generally facing the patient's femoral artery.

In the preferred embodiment, the cover is slidable over the container element, the method including the step of placing the assembly adjacent a patient and sliding the cover over the patient.

### Brief Description of the Drawings

Embodiments of the present invention are described below, by way of example only, with reference to the accompanying drawings, in which:
Figure 1 shows an example of a typical introducer assembly;
Figure 2 is a schematic diagram in perspective of a preferred embodiment of container assembly;
Figure 3 is a front view of the cover of the assembly of Figure 2;
Figure 4 is a cross-sectional view in side elevation of the assembly of Figure 2; and
Figure 5 is a schematic view of the assembly of Figure 2 in use.

### Description of the Preferred Embodiments

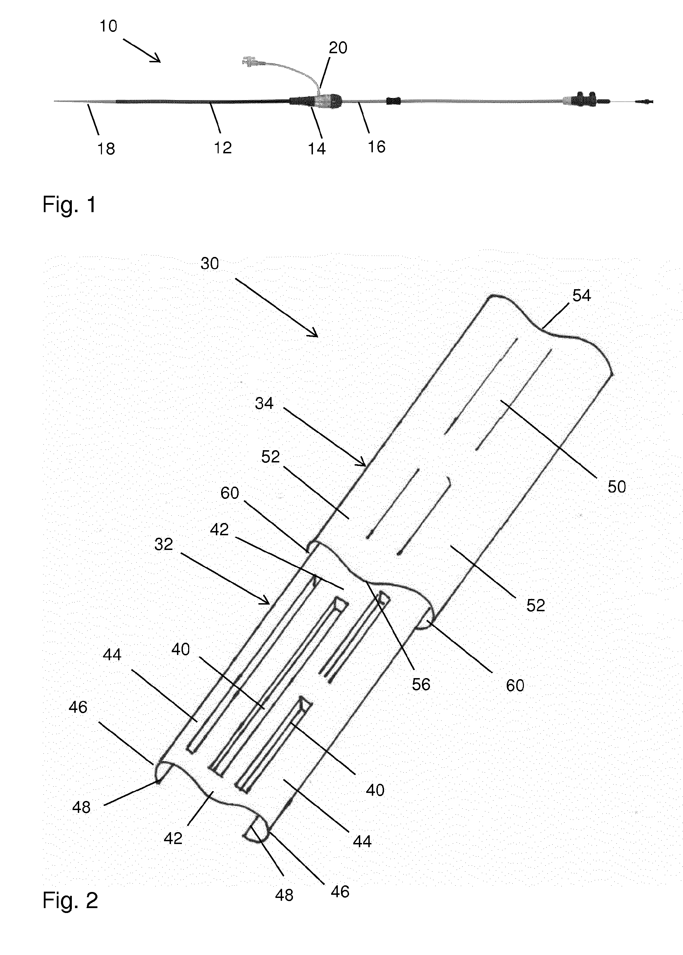

Referring first to Figure 1, this shows an example of an introducer assembly 10 used for delivering an implantable medical device into a patient by endoluminal delivery. The assembly 10 typically includes a sheath 12 fixed to a handle and haemostatic valve unit 14. Disposed within the sheath 12 in Figure 1 is a delivery catheter 16 terminating with a dilator tip 18 at its distal end. The assembly 10 is typically fed into the patient from a percutaneous entry point, usually over a pre-inserted guide wire (not shown). A conventional deployment procedure may include the following steps:
1) upon removal of the individual components of the assembly from the packaging, using the side arm 20 of the valve 14, the assembly 10 is flushed with heparinized saline solution;
2) flush the dilator 18 with heparinized solution;
3) insert the dilator 18 completely into the introducer sheath 12,
4) as appropriate wet the outer surface of the assembly 10 with a hydrophilic coating;
5) using the standard Seldinger technique access the target vessel with an appropriate needle;
6) insert a guide wire into the vessel through the needle, then remove the needle, leaving the guide wire in place;
7) inert the dilator 18 and sheath 12 assembly over the guide wire and feed endoluminally until the distal end of the sheath has reached the site of treatment;
8) remove the guide wire and dilator 18, then aspirate and flush the sheath 12 through the side arm 20;
9) insert into the sheath 12 the appropriate device as needed, for instance implantable medical device carrier, medical or diagnostic tool;
10) deploy the medical device or utilise the tool;
11) remove the device carrier or tool;
12) remove the sheath.
There are therefore several steps involved in the medical process and various different components.

The sheath 12 can be of significant length, for instance well over one metre when introduced via the femoral artery. It will be appreciated that during the procedure there will be times when the physician will wish to release grip of the handle and valve unit 14, for instance to prepare another part of the device or for performing another step of the procedure. This can be difficult though without losing the desired position or orientation of the proximal part of the assembly 10.

Referring now to Figure 2, this shows in schematic form a preferred embodiment of container assembly 30 for storing and holding in position apparatus of the type shown in Figure 1. The container assembly 30 includes a base or container element 32 for storing the surgical components and a cover 34 which fits over the base element 32. The base element 32 is in this embodiment formed of sheet material, typically a suitable plastics material, which is molded into the shape shown. In particular, the base element 34 includes a plurality of recesses or chambers 40 therein, each shaped and sized to hold securely an associated element of the surgical apparatus. For example, one of the chambers 40 may hold the guide wire, another chamber 40 may hold the sheath, and so on. The skilled person will appreciate that although Figure 2 shows the chambers 40 being rectangular, these will have shapes and sizes to correspond to the packaged shape of the associated medical item or tool. For instance, one or more of the chambers 40 may be round or annular to hold a coiled guide wire. The number, the sizes and shapes of the chambers 40 is not important to the teachings herein.

The base element 32 has a generally elongate shape, in this example being generally rectangular in plan, with first and second ends and long sides. At its upper surface, the base element includes a rounded depression or channel 42 which extends the whole length of the base element 32 as will be apparent in the drawings. Either side of the channel 42, the upper surface of the base element 32 provides rounded ribs or ridges 44, which also extend for the whole length of the base element 32. The chambers 40, it will be appreciated, will be open at the upper surface of the base element and will therefore follow the curved contour of this surface. Given the varying height of the upper surface of the base element, the depth of the chambers 40 will vary and in any event will be consistent with the thickness or depth of the associated components to be held in the chambers 40.

The base element 32 preferably has rounded sides 46, which in the embodiment shown curve inwardly to form bottom support surfaces 48 which in practice provide the lower support surface of the base element 32.

Although in the embodiment of Figure 2 the base or container element 32 is formed from a sheet material suitably molded, it is to be understood that it could be a solid structure formed to have a curved upper surface consistent with the curves 42, 44 and rounded sides 46. In one example, the base element could be formed from expanded polystyrene. It is preferred, though, that the base element 32 is formed of sheet material due to advantages of reduced weight and improved disposability.

The cover or lid 34 of the container assembly 30 is also formed of sheet material in this embodiment and has a shape which conforms to the curved contours of the base element 32. In particular, the cover 34 includes a central recess or channel 50 which in this embodiment extends for the length of the cover, that is to its proximal 56 and distal 54 ends so as to be open at these ends. In other embodiments, the channel 50 may be closed at the proximal end 56, that is the end which remains in overlying relationship with the base element 32.

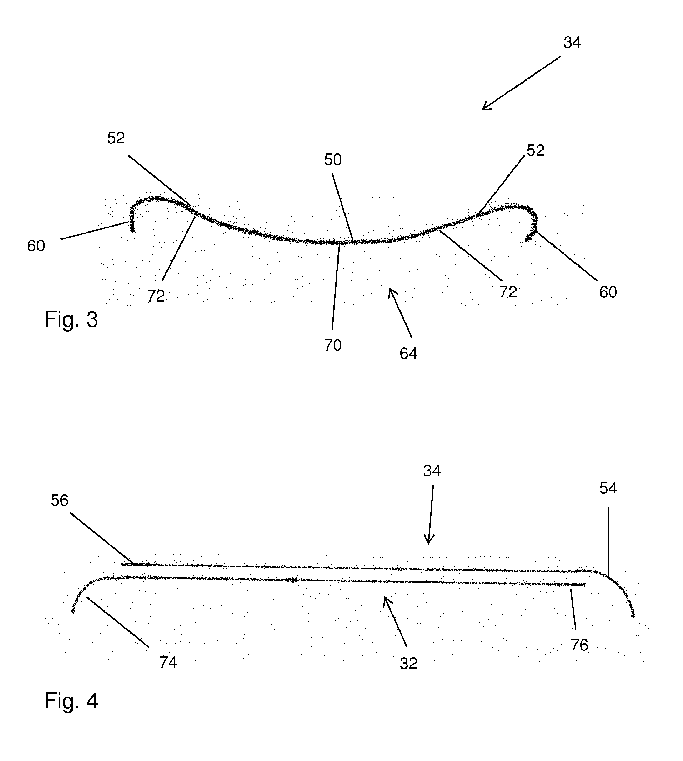

The cover 34 also includes two rounded ribs 52 which lies either side of the channel 50 and have a shape and size consistent with the shape and size of the longitudinal ribs 44 of the base element 32. The rounded ribs 52 extend to rounded sides 60 which again conform to the shape and dimensions of the rounded sides 46 of the base element 32. As a result of the sheet form of the cover 34, this has a lower surface of a shape equivalent to but inversed relative to its upper surface shape, as will be apparent from Figure 3.

Specifically, the lower or inner surface 64 of the cover 34 includes an elongate central convex region 70 extending for the length of the cover 34 or at least that part of the cover 34 which in practice extends beyond the base element 32 when the casing 10 is open. Either side of the rounded convex region 70 are elongate concave zones or channels 72 which extend in the same manner as the convex region 70. The rounded sides 60 of the cover 34 curve inwardly in this embodiment, in practice to provide a secure coupling between the cover 34 and base element 32 such that these cannot be separated by pulling the cover 34 upwardly. In other embodiments, though, the rounded sides 60 terminate short of curving inwardly, for patient comfort as explained below.

The cover 34 may have the shape described above for the whole of its length. However, in some embodiments, the cover 34 may have a slightly different shape at its proximal end 56 or distal end 54. An example is shown in Figure 4, which is a schematic diagram of the container assembly 10 taken in cross-section along its longitudinal direction. The distal end 54 of the cover 34 in this embodiment curves downwardly, towards the base element 32, and provides a rounded front end to the assembly 10. The proximal end of the cover 34, on the other hand, is straight with the main portion of the cover 34, enabling the cover 34 to slide over the base element 32.

The base element 32 includes, in this embodiment, a straight distal end 76, although this could be rounded in the same manner as the distal end 54 of the cover 34. The proximal end 74 of the base element 32 is curved in a manner similar to the curvature of the distal end 54 of the cover 34. Thus, both ends of the assembly 10, one provided by the distal end 54 of the cover 34 and the other provided by the proximal end 74 of the base element 32, are rounded. This, coupled to the rounding of the sides of the assembly 10, ensures that there are no sharp corners to the assembly 10 which could inconvenience the user or contribute to increased risk of damage to the contents in the assembly 10 in the event that the assembly 10 is dropped or knocked during transportation, storage or use.

The cover 34 is slidable relative to the base 32 and able to sit over the base 32 to close the latter and therefore seal any medical components held in the container assembly 10. The cover 34 may be sealable to the base 32 so as to retain sterile integrity of the medical components, although in other embodiments there may be provided a wrapping over the container assembly, for instance in the form of a bag, which wrapping maintains the sealed sterile environment.

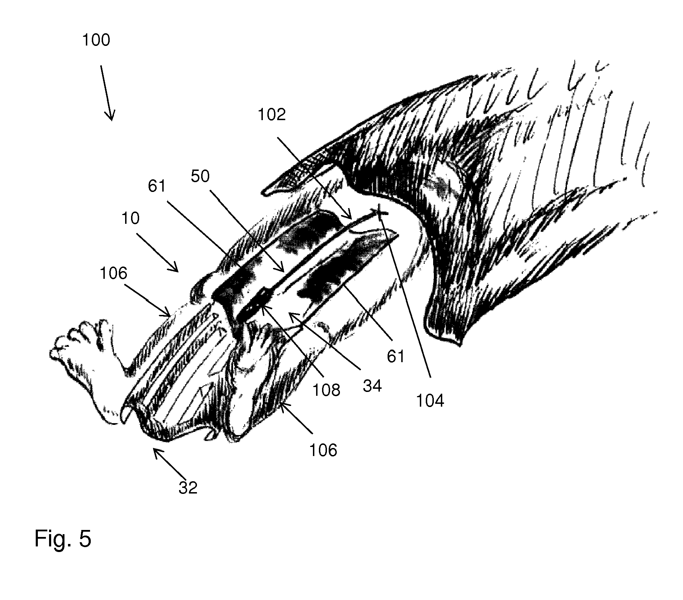

The cover 34 is also slidable in a distal direction so as to expose the contents of the assembly 10, that is to expose the chambers 40. In this regard, the cover 34 will preferably remain attached to the base 32, in this embodiment by means of the inwardly extending sides 60. Where the cover 34 has a flatter shape, that is does not have inwardly extending sides 60 as in the embodiment of Figure 5 described below, the cover could be attached to the base 32 by other means, for instance by a sliding coupling between the cover 34 and base 32. A suitable sliding coupling will be apparent to the skilled person and may, for example, be in the form of a guide channel on one of the base 32 and cover 34 and an associated guide element on the other of the base 32 and cover 34. In other embodiments, the cover 34 need not be attached to the base 32 after being opened and may just rest on a part of the base 32.

Referring now to Figure 5, this shows an implementation of the invention for deployment of medical apparatus through the femoral artery of a patient 100. In the example in this Figure, the cover 34 has sides 61 which are substantially parallel with the plane of the cover 34, that is do not curve inwardly as in the embodiment of Figures 2 to 4, and have no or only minimal downward curvature, in particular a downward curvature of no greater than about the curvature of a patient's thighs. In all other respects, the container assembly 30 of Figure 5 has the characteristics of all the other embodiments disclosed herein.

The case assembly 30 has been positioned between the patient's legs 106 and is evidently sized and shaped to fit. A medical introducer assembly 102, which would have been stored in the chambers 40 of the base element 32, can be partially inserted into the patient through a percutaneous entry point 104, in a manner known in the art. The proximal end 108 of the introducer assembly 102 remains outside the patient and can be seen resting on the casing assembly 30 and in particular in the channel 50 of the cover 34. The shape of the channel ensures that the proximal end 108 of the introducer assembly 102 remains in position adjacent the patient and importantly aligned with the percutaneous entry point 104.

The cover 34 can be seen extending beyond the base 32 and resting on the patient's thighs. The shape of the lower surface of the cover 34, in particular of the rounded concave channels 72 in the lower surface, ensures a good and comfortable fit on the patient's thighs. This arrangement also makes it is possible to move the cover 34 closer to the percutaneous entry point 34 and thus to provide for close support of the introducer assembly 102, without having to modify heavily the shape of the casing assembly 10.

The cover and in particular its contoured top surface with its channel 50 provides a convenient rest element for an introducer assembly or other medical tool, freeing the physician to concentrate of the other aspects of the medical procedure without having to be concerned as to maintaining the correct position or orientation of the device temporarily inserted into the patient.

The example of Figure 5, being for a femoral introducer assembly, may have a length of 1000 to 1200 mm or more. In particular, it is preferred that the container assembly 10 has a height, when on a support surface such as an operating table, the same as or similar to the percutaneous entry point and in practice to the height of a person's thighs when prostrate. The channel 50 need not have dimensions equivalent to the size of the handle 108 of the introducer assembly as it has the function of holding this in the correct orientation, which is can achieve by way of its shape.

The casing assembly 10 may be made of a variety of suitable materials, including for example: PET or PETE (polyethylene terephthalate). The skilled person will be aware of other suitable materials.

In the embodiments described above and shown in the drawings the channel 50 extends in the direction of the longitudinal axis of the casing apparatus 30. In other embodiments, though, the channel 50 could extend at an angle to the longitudinal axis and typically at an angle suitable to cause the channel 50 to point towards the intended percutaneous entry point. In the example of Figure 5, for instance, the channel 50 could be oriented towards the femoral artery. It will be appreciated that a different orientation of the channel 50 will affect the shape and size of the ridges 52 and the corresponding features on the base element 32 will be likewise modified.

Similarly, although the embodiments described above has a channel 50 which is straight, it could be curved in some embodiments.

It is also envisaged that the channel 50 could have a tapering shape in transverse cross-section and not necessarily rounded. In some embodiments, for instance, it could have generally straight side walls tapering to a narrow base.

In the embodiments described above, the cover 34 is made of sheet material having a substantially uniform thickness. Other embodiments could produce the curvature of the top surface of the cover 34 by changes in thickness of the cover, in which case the lower surface of the cover 34 and the upper surface of the base element 32 could have a different shape and even be flat. This is not necessarily preferred, though, for reasons of economy in the amount of material used for the assembly 30.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosure in the abstract accompanying this application are incorporated herein by reference.

## Claims

**1.** A surgical apparatus container assembly including a container element provided with a plurality of receptacles for accommodating surgical apparatus; and a cover shaped to fit over the container element and to close said receptacles, the cover including an upper surface forming an outer surface of the assembly, the upper surface including an elongate channel extending at least partially along the upper surface and being open at an end of the upper surface; the elongate channel being rounded or tapering in transverse cross-section.

**2.** An assembly according to claim 1, wherein the elongate channel extends along the entire of the upper surface and is open at both of opposing ends of the assembly.

**3.** An assembly according to claim 1 or 2, wherein the cover has a length which extends in a direction of a longitudinal axis of the assembly, the elongate channel extending substantially parallel or at an angle to said longitudinal axis.

**4.** An assembly according to any preceding claim, wherein the cover includes a lower surface, which lower surface is provided with one or more elongate channels extending at least partially along the lower surface.

**6.** An assembly according to claim 5, wherein the cover includes two elongate channels extending at least partially along the lower surface.

**7.** An assembly according to claim 6, wherein the two elongate channels are disposed either side of the elongate channel in the upper surface.

**8.** An assembly according to claim 5, 6 or 7, wherein the or each elongate channel is rounded in transverse cross-section.

**9.** An assembly according to any one of claims 5 to 8, wherein the or each elongate channel is sized to accommodate a part of a patient's body.

**10.** An assembly according to claim 9, wherein the or each elongate channel is sized to rest on a patient's leg.

**11.** An assembly according to any preceding claim, wherein the cover is formed from sheet material having a generally uniform thickness.

**12.** An assembly according to any preceding claim, wherein the container element has an upper surface, which upper surface has a shape conforming to or similar to the upper surface of the cover.

**13.** An assembly according to any preceding claim, wherein the cover is slidable over the container element.

**14.** An assembly according to claim 13, including guide elements on the cover and container element for guiding the cover in sliding manner over the container element and shaped to prevent pulling apart of the cover and container element from one another.

**15.** An assembly according to claim 14, wherein the guide elements include rounded depending edges to the cover and corresponding rounded side walls to the container element.

**16.** An assembly according to claim 13, 14 or 15, wherein the cover is slidable over the container element substantially in the direction of the elongate channel.

**17.** An assembly according to any preceding claim, wherein the cover and container element are sealable to one another, thereby to seal the receptacles.
